# EUROPEAN PATENT SPECIFICATION

(11) **EP 2 952 146 B1**
(45) Date of publication and mention of the grant of the patent: **30.12.2020**
(21) Application number: 15170430.1
(22) Date of filing: 03.06.2015
(51) Int. Cl.: A61B 17/34, A61B 17/00

(54) **TROCARS AND OBTURATORS**
TROKARE UND OBTURATOREN
TROCARTS ET OBTURATEURS

(30) Priority: 06.06.2014 US 201414298149
(43) Date of publication of application: 09.12.2015
(73) Proprietor: SurgiQuest, Inc., Milford, CT 06460 (US)
(72) Inventor: Mastri, Dominick, Bridgeport, Connecticut 06606 (US); Augelli, Michael, Prospect, Connecticut 06712 (US); Kane, Michael J., Clinton, Connecticut 06413 (US)
(74) Representative: Hoefer & Partner Patentanwälte mbB

(56) References cited:
- WO-A1-2015/112484
- CA-A1- 2 852 668
- US-A- 6 017 356
- US-A1- 2003 045 834
- US-A1- 2005 010 238
- US-A1- 2008 009 797
- US-A1- 2008 161 720
- US-A1- 2008 249 475
- US-A1- 2010 298 857
- US-A1- 2011 040 149
- US-A1- 2011 237 901
- US-A1- 2011 319 826

## Description

### BACKGROUND OF THE INVENTION

### 1. Field of the Invention

The present disclosure relates to medical devices, and more particularly to trocars and obturators for use in laparoscopic and percutaneous surgical procedures, for example.

### 2. Description of Related Art

Laparoscopic, or "minimally invasive" surgical techniques have become common. Benefits of such procedures include reduced trauma to the patient, reduced opportunity for infection, and decreased recovery time. Such procedures within the abdominal cavity are typically performed through a device known as a trocar or cannula, which facilitates the introduction of laparoscopic instruments into the abdominal cavity of a patient.

Additionally, such procedures commonly involve filling or "insufflating" the abdominal (peritoneal) cavity with a pressurized fluid, such as carbon dioxide, to create what is referred to as a pneumoperitoneum. The insufflation can be carried out by a trocar equipped to deliver insufflation fluid, or by a separate insufflation device, such as an insufflation needle. Introduction of surgical instruments into the pneumoperitoneum without a substantial loss of insufflation gas is desirable, in order to maintain the pneumoperitoneum. During typical laparoscopic procedures, a surgeon makes three to four small incisions, usually no larger than about twelve millimeters each, which are typically made with the trocar devices themselves, typically using a separate inserter or obturator placed therein. Following insertion, the inserter is removed, and the trocar allows access for instruments to be inserted into the abdominal cavity.

The trocar can provide a means to maintain the pressure within the cavity, so that the surgeon has an open interior space in which to work, by sealing between the trocar and the surgical instrument being used, while still allowing at least a minimum freedom of movement of the surgical instruments. Such instruments can include, for example, scissors, grasping instruments, occluding instruments, cauterizing units, cameras, light sources and other surgical instruments.

Such conventional methods and systems have generally been considered satisfactory for their intended purpose.

US 2008/161720 A1 discloses a medical targeting and device introduction system that includes an introducer having a cannula, a hemostatic valve and a hub. The cannula is defined, at least in part, by an inner lumen. The hub includes a latch to releasably secure the hub to a biopsy device. The hub includes a proximal end and a distal end. The latch extends from the proximal end, and the hemostatic valve is interposed within the introducer.

CA 2 852 668 A1 discloses an optical trocar system that includes an elongated obturator member having a first diameter. The elongated obturator member has a distal region, the distal region having a first diameter section having a diameter that is substantially equal to the first diameter of the elongated obturator member. The elongated obturator member has a second diameter section having a diameter that is less than the first diameter. The optical trocar system also includes an optical member attached to the distal region of the elongated obturator member such that the optical member encapsulates the second diameter section of the elongated obturator member and at least a portion of the first diameter section. A longitudinal bore through the elongated tubular obturator member is configured to receive an endoscope.

WO 2015/112484 A1, which is a document that falls under Art. 54(3) EPC, discloses a medical device for accessing body tissue or fluid. The medical device may include an introducer sheath and a valve that impedes the flow of fluid through the introducer sheath. WO 2015/112484 A1 further discloses an assembly for selectively coupling components of a medical device. The assembly may comprise two components configured to engage with one another by at least a snap fit-type connection. This connection may facilitate disengagement of the two components via actuation of a release mechanism.

However, there is still a need in the art for improved trocars, obturators, and the like. The present disclosure provides a solution for this need.

### SUMMARY

The solution for the aforementioned need is provided by the subject matter of the independent claim 1, which also defines the present invention.

A trocar assembly includes a trocar including an elongated tubular member extending between a distal end configured to be inserted into a surgical site and a proximal end including a housing configured for introduction of surgical instruments into the tubular member. The housing includes a pair of opposed latch receptacles. An obturator is assembled into the tubular member of the trocar. The obturator includes an elongated obturator body extending from a trocar cover to an opposed obturator tip. The trocar cover includes a single rim for engaging the housing of the trocar. The trocar cover includes a pair of opposed latches extending radially inward from the rim, wherein the latches are releasably latched to the latch receptacles of the trocar. The trocar cover includes a compliant mechanism operatively connected to release the latches from the latch receptacles.

The compliant mechanism includes a pair of opposed pinch regions circumferentially offset from the latches. The compliant mechanism is configured to move the latches from latched positions to release positions radially outward from the latched positions upon pinching the pinch regions toward one another. Each of the pinch regions can include a textured surface to facilitate actuation of the compliant mechanism. The trocar cover can include an anti-rotation key engaged to an anti-rotation key receptacle defined in the housing of the trocar to resist relative rotation of the trocar and obturator.

The obturator tip can include a latch receptacle and an anti-rotation key, wherein the obturator body includes a latch engaged in the latch receptacle of the obturator tip, and wherein the obturator body includes an anti-rotation key receptacle engaged to the anti-rotation key of the obturator tip.

The obturator tip can include a second anti-rotation key opposed to the first anti-rotation key and the obturator body can include a second anti-rotation key receptacle opposed to the first anti-rotation key receptacle. The first and second anti-rotation keys can be engaged to the first and second anti-rotation key receptacles, respectively.

The obturator tip can include a second latch receptacle opposed to the first latch receptacle of the obturator tip. The obturator body can include a second latch opposed to the first latch of the obturator body. The first and second latches of the obturator body can be engaged to the first and second latch receptacles of the obturator tip, respectively.

The obturator tip can include an engagement member extending into and engaged with an interior bore of the obturator body. The engagement member can include a hard stop surface blocking a radially outward portion of the interior bore for providing a hard stop to endoscopes moving within the interior bore. The engagement member can be sealingly engaged to the interior bore of the obturator body to prevent leakage between the obturator body and the obturator tip. The engagement member can include a full annular sealing surface sealed to the interior bore of the obturator body, e.g. proud of the latches and anti-rotation keys.

These and other features of the systems of the subject disclosure will become more readily apparent to those skilled in the art from the following detailed description of the preferred embodiments taken in conjunction with the drawings.

### BRIEF DESCRIPTION OF THE DRAWINGS

So that those skilled in the art to which the subject disclosure appertains will readily understand how to make and use the devices and methods of the subject disclosure without undue experimentation, preferred examples thereof will be described in detail herein below with reference to certain figures, wherein:
Fig. 1 is a perspective view of an exemplary embodiment of a trocar assembly constructed in accordance with the present disclosure, showing the obturator assembled into the trocar;
Fig. 2 is a perspective view of the obturator of Fig. 1, showing the obturator removed from the trocar;
Fig. 3 is a perspective view of the trocar cover of the obturator of Fig. 2, schematically indicating the latch release action of the compliant mechanism when the pinch points are pinched together;
Fig. 4 is a perspective view of a portion of the trocar assembly of Fig. 1, showing the obturator unlatched from the trocar;
Fig. 5 is a perspective view of a portion of the trocar assembly of Fig. 4, showing the anti-rotation key and corresponding receptacle of the housing of the trocar;
Fig. 6 is a perspective view of a portion of the obturator of Fig. 2, showing the opposed latches extending radially inward from the rim of the trocar cover;
Fig. 7 is a perspective view of a portion of the obturator of Fig. 2, showing the trocar cover from a view point generally opposite of that of Fig. 6;
Fig. 8 is a partially cut away exploded perspective view of a portion of the obturator of Fig. 2, showing the obturator tip exploded away from the obturator body;
Fig. 9 is a cross-sectional side elevation view of the obturator of Fig. 8, showing the obturator tip assembled onto the obturator body;
Fig. 10 is an exploded perspective view of another exemplary embodiment of an obturator constructed in accordance with this disclosure, showing a three piece obturator construction;
Fig. 11 is a cross-sectional side elevation view of the obturator of Fig. 10, showing the connection between the obturator body and the trocar cover; and
Fig. 12 is an exploded perspective view of the obturator of Fig. 10, showing another exemplary obturator tip in place of the one shown in Fig. 10.

### DETAILED DESCRIPTION OF THE PREFERRED EMBODIMENTS

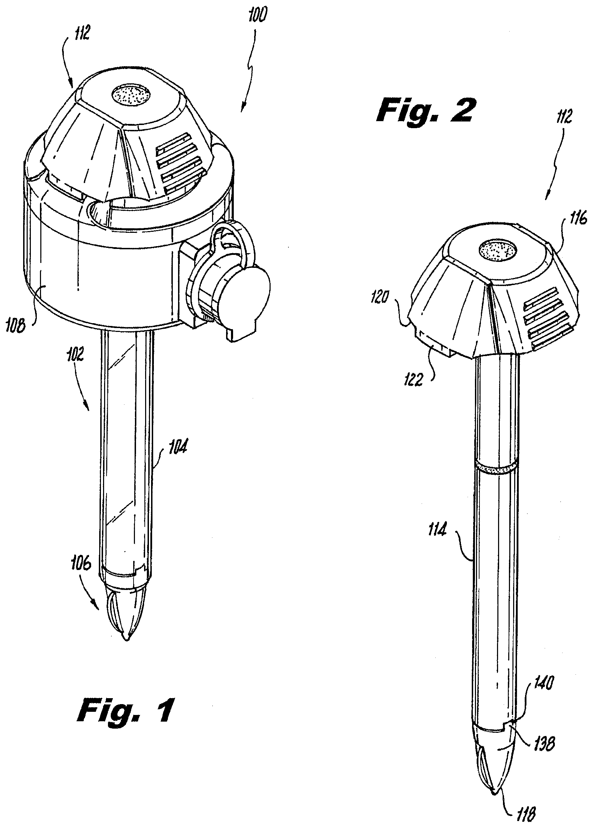

Reference will now be made to the drawings wherein like reference numerals identify similar structural features or aspects of the subject disclosure. For purposes of explanation and illustration, and not limitation, a partial view of an exemplary embodiment of a trocar assembly in accordance with the disclosure is shown in Fig. 1 and is designated generally by reference character 100. Other embodiments of trocar assemblies in accordance with the disclosure, or aspects thereof, are provided in Figs. 2-9, as will be described. The systems and methods described herein can be used for laparoscopic and percutaneous procedures, for example.

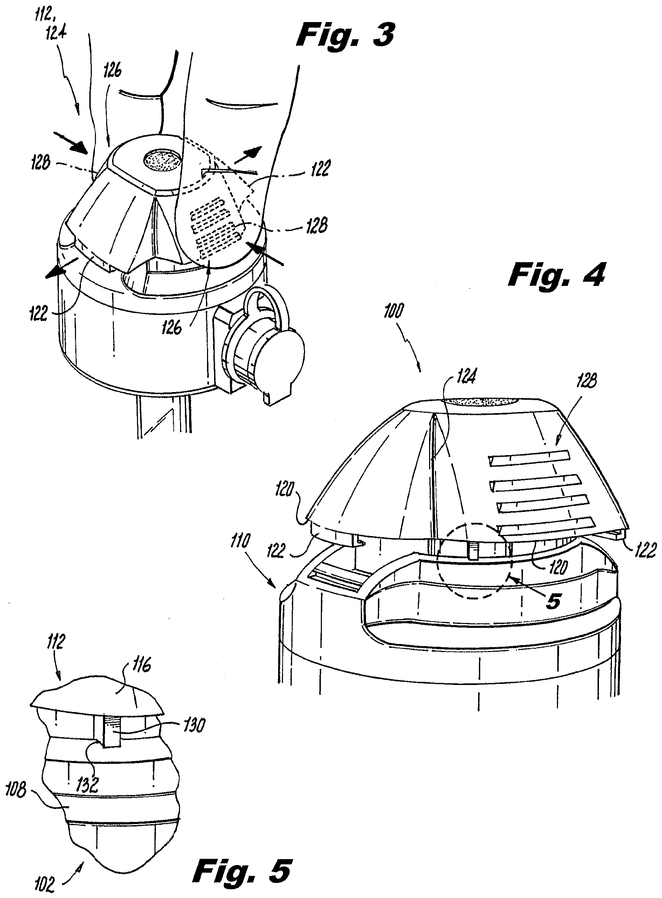

Trocar assembly 100 includes a trocar 102 including an elongated tubular member 104 extending between a distal end 106 configured to be inserted into a surgical site and a proximal end including a housing 108 configured for introduction of surgical instruments into the tubular member 104. Housing 108 includes a pair of opposed latch receptacles 110, one of which is shown in Fig. 4. An obturator 112 is assembled into the tubular member 104 of trocar 102.

Referring now to Fig. 2, obturator 112 includes an elongated obturator body 114 extending from a trocar cover 116 to an opposed obturator tip 118. Trocar cover 116 includes a rim 120 for engaging the housing 108 of trocar 102. Trocar cover 116 includes a pair of opposed latches 122 extending radially inward from rim 120, wherein the latches 122 are releasably latched to the latch receptacles 110 of the trocar. Trocar cover 116 includes a compliant mechanism 124 operatively connected to release the latches 122 from the latch receptacles 110.

With reference now to Fig. 3, the compliant mechanism 124 includes a pair of opposed pinch regions 126 circumferentially offset from the latches 122. The compliant mechanism 124 is configured to move the latches 122 from their latched positions to release positions radially outward from the latched positions upon pinching the pinch regions toward one another. The release position is indicated schematically with the outward pointing arrows in Fig. 3. The pinching motion, e.g., manual pinching by a surgeon, is indicated schematically by the fingers and inward pointing arrows in Fig. 3, and the resultant outward motion of latches 122 is indicated schematically in Fig. 3 with the outward pointing arrows. Each of the pinch regions 126 includes a textured surface 128 to facilitate actuation of the compliant mechanism 124.

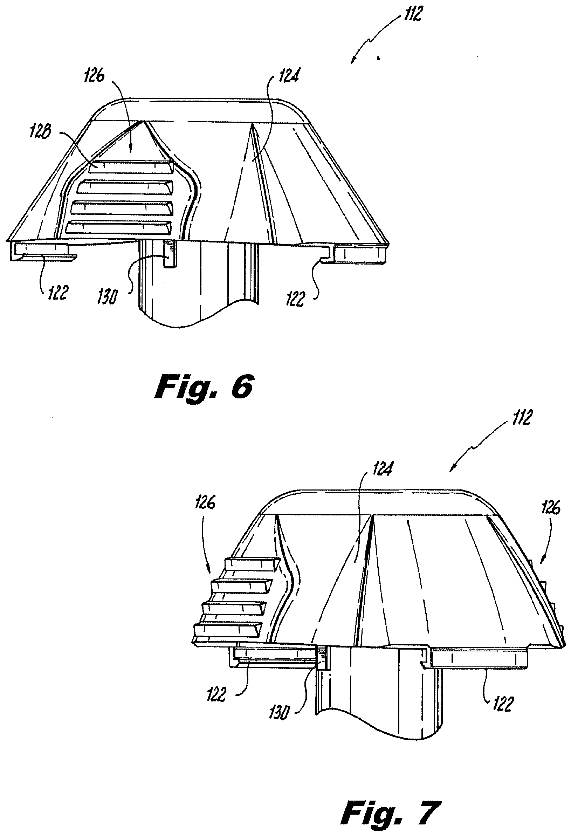

Referring now to Figs. 4 and 5, trocar assembly 100 is shown prior to latching or after unlatching obturator 112 from trocar 102. Fig. 4 shows trocar assembly 100 with latches 122 of obturator 112 released from housing 108. Trocar cover 116 includes an anti-rotation key 130 engaged to an anti-rotation key receptacle 132 defined in housing 108 of trocar 102 to resist relative rotation of the trocar 102 and obturator 112, as shown in Fig. 5. Two opposed anti-rotation keys 130 are included in obturator 112, as shown in Figs. 6-7. Figs. 6-7 also show both of the latches 122 extending radially inward from rim 120.

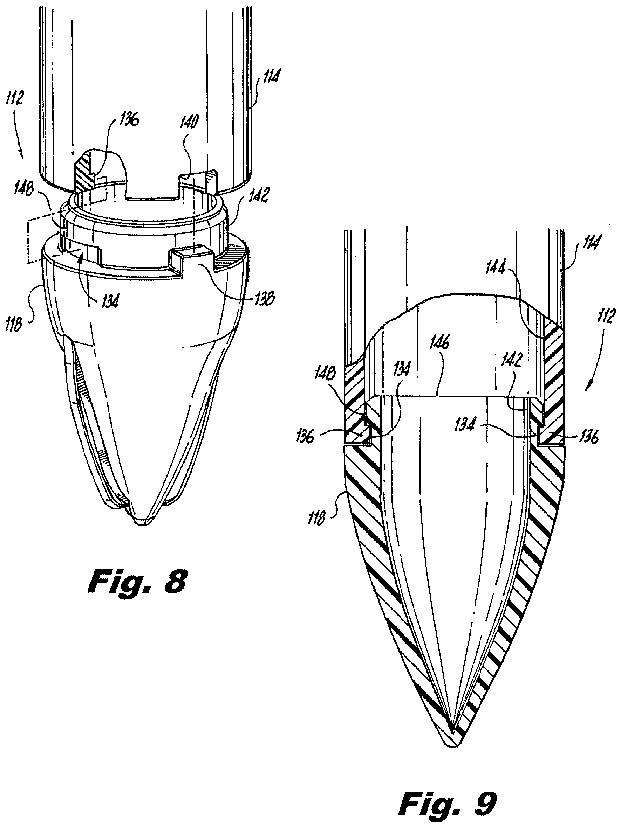

With reference now to Figs. 8-9, obturator tip 118 is described in greater detail. Obturator tip 118 includes a pair of opposed latch receptacles 134. Obturator body 114 includes a pair of opposed latches 136, each engaged in the respective latch receptacle 134 of the obturator tip, as shown in Fig. 9.

Obturator tip 118 also includes a pair of diametrically opposed anti-rotation keys 138 circumferentially offset from the latch receptacles 134 by 90°. Obturator body 114 includes a pair of diametrically opposed anti-rotation key receptacles 140 each engaged to a respective one of the anti-rotation keys 138. While only one anti-rotation key and receptacle are shown in Fig. 8, the other side is shown in Fig. 2.

Obturator tip 118 includes an annular engagement member 142 extending into and engaged with interior bore 144 of obturator body 114. Engagement member 142 includes a hard stop surface 146 blocking a radially outward portion of interior bore 144 for providing a hard stop to endoscopes moving within interior bore 144. Engagement member 142 is sealingly engaged to the surface of interior bore 144 to prevent leakage between obturator body 114 and obturator tip 118. Engagement member 142 includes a full annular sealing surface 148 sealed to interior bore 144, e.g. proud of the latches 136 and anti-rotation keys 138 as oriented in Fig. 8.

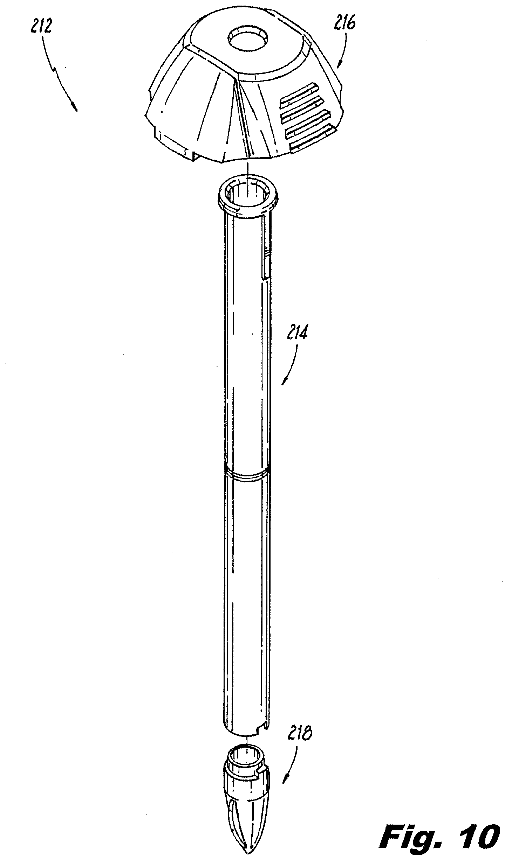

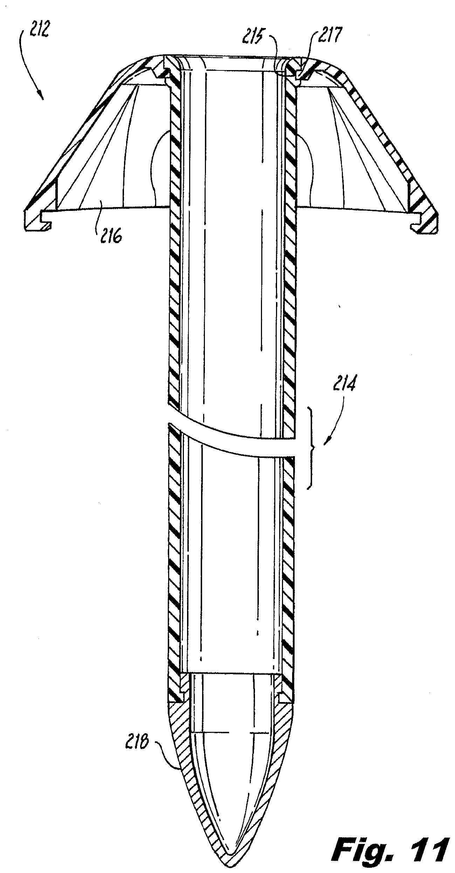

In obturator 112, obturator body 114 is integral with trocar cover 116. It is also contemplated that these can be made as separate pieces joined together, as in obturator 212 in Fig. 10. Obturator 212 includes an obturator tip 218 that is attached to obturator body 214 in the same manner described above with respect to obturator tip 118. Trocar cover 216 is a separate piece that can be attached to obturator body 214 as shown in Fig. 11. Lip 217 of trocar cover 216 seats in detent 215 of obturator body 214. This allows trocar cover 216 and obtrurator body 214 to be made of two different materials. For example, trocar cover 216 can be made of a relatively flexible material to facilitate actuation of the latches as described above, and obturator body 214 can be made of a relatively rigid material to facilitate introduction to a surgical site.

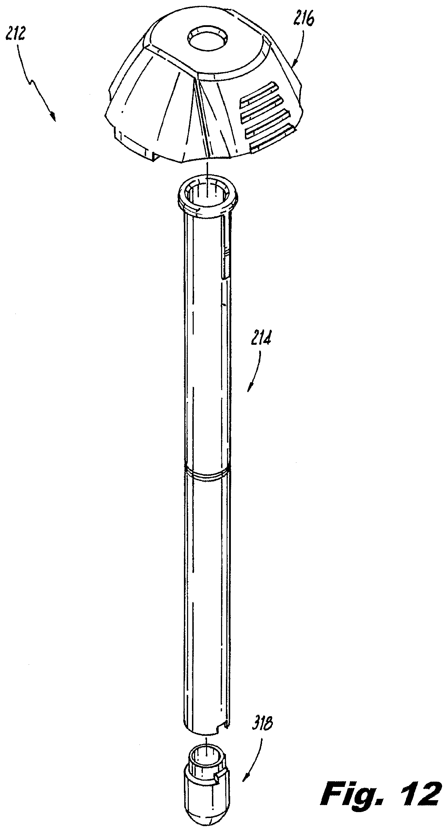

With reference to Fig. 12, obturator 212 is shown with another exemplary embodiment of an obturator tip, namely obturator tip 318, which is generally blunt relative to obturator tips 118 and 218 described above. Those skilled in the art will readily appreciate that any other suitable tip geometry can be used as needed on an application by application basis without departing from the scope of this disclosure.

The methods and systems of the present disclosure, as described above and shown in the drawings, provide for trocars and obturators with superior properties including improved assembly and ease of use.

## Claims

1. An obturator (112) comprising:
an elongated obturator body (114) extending from a trocar cover (116) to an opposed obturator tip (118), wherein the trocar cover (116) includes a single rim (120) for engaging a trocar (102), wherein the trocar cover (116) includes a pair of opposed latches (122) extending radially inward from and extending distally from the rim (120), wherein the trocar cover (116) includes a compliant mechanism (124) operatively connected to move the latches (122) between latched positions and release positions radially outward from the latched positions, wherein the compliant mechanism (124) includes a pair of opposed pinch regions (126) circumferentially offset from the latches (122) around the single rim (120), wherein the compliant mechanism (124) is configured to move the latches (122) from the latched positions to the release positions radially outward from the latched positions upon pinching the pinch regions (126) toward one another.

2. The obturator (112) as recited in claim 1, wherein the obturator tip (118) includes a latch receptacle (134) and an anti-rotation key (138), and wherein the obturator body (114) includes a latch (136) engaged in the latch receptacle (134), and wherein the obturator body (114) includes an anti-rotation key receptacle (140) engaged to the anti-rotation key (138).

3. The obturator (112) as recited in claim 1 or 2, wherein each of the pinch regions (126) includes a textured surface (128) to facilitate actuation of the compliant mechanism (124).

4. The obturator (112) as recited in any of the preceding claims, wherein the obturator tip (118) includes an engagement member (142) extending into and engaged with an interior bore (144) of the obturator body (114), wherein the engagement member (142) includes a hard stop surface (146) blocking a radially outward portion of the interior bore (144) for providing a hard stop to endoscopes moving within the interior bore (144).

5. The obturator (112) as recited in claim 4,
- wherein the engagement member (142) is sealingly engaged to the interior bore (144) of the obturator body (114) to prevent leakage between the obturator body (114) and the obturator tip (118), and/or
- wherein the engagement member (142) includes a full annular sealing surface (148) sealed to the interior bore (144) of the obturator body (114).

6. The obturator (112) as recited in any of claims 2 to 5,
- wherein the latch receptacle (134) is a first latch receptacle, wherein the obturator tip (118) includes a second latch receptacle (134) opposed to the first latch receptacle (134), wherein the latch (136) is a first latch, wherein the obturator body (114) includes a second latch (136) opposed to the first latch (136), wherein the first and second latches (136) are engaged to the first and second latch receptacles (134), respectively, and/or
- wherein the anti-rotation key (138) is a first anti-rotation key, wherein the obturator tip (118) includes a second anti-rotation key (138) opposed to the first anti-rotation key (138), wherein the anti-rotation key receptacle (140) is a first anti-rotation key receptacle, wherein the obturator body (114) includes a second anti-rotation key receptacle (140) opposed to the first anti-rotation key receptacle (140), wherein the first and second anti-rotation keys (138) are engaged to the first and second anti-rotation key receptacles (140), respectively.

7. A trocar assembly (100) comprising:
a trocar (102) including an elongated tubular member (104) extending between a distal end (106) configured to be inserted into a surgical site and a proximal end including a housing (108) configured for introduction of surgical instruments into the tubular member (104), wherein the housing (108) includes a pair of opposed latch receptacles (110); and
an obturator (112) as recited in any of the preceding claims, wherein the latches (122) of the obturator (112) are releasably latched to the latch receptacles (110) of the trocar (102), wherein the compliant mechanism (124) is operatively connected to release the latches (122) from the latch receptacles (110).

8. The trocar assembly (100) as recited in claim 7, wherein the trocar cover (116) includes an anti-rotation key (130) engaged to an anti-rotation key receptacle (132) defined in the housing (108) of the trocar (102) to resist relative rotation of the trocar (102) and obturator (112).

## Patentansprüche

1. Obturator (112), umfassend:
- einen länglichen Obturatorkörper (114), der sich von einer Trokarabdeckung (116) zu einer gegenüberliegenden Obturatorspitze (118) erstreckt, wobei die Trokarabdeckung (116) einen einzelnen Rand (120) zum Eingreifen in einen Trokar (102) umfasst, wobei die Trokarabdeckung (116) ein Paar von gegenüberliegenden Verriegelungen (122) umfasst, die sich radial vom Rand (120) nach innen und distal von diesem erstrecken, wobei die Trokarabdeckung (116) einen konformen Mechanismus (124) umfasst, der funktionsmäßig verbunden ist, um die Verriegelungen (122) zwischen den Verriegelungspositionen und Freigabepositionen radial nach außen von den Verriegelungspositionen zu bewegen, wobei der konforme Mechanismus (124) ein Paar von gegenüberliegenden Klemmbereichen (126) umfasst, die in Umfangsrichtung von den Verriegelungen (122) um den einzelnen Rand (120) versetzt sind, wobei der konforme Mechanismus (124) eingerichtet ist, um die Verriegelungen (122) von den verriegelten Positionen zu den Freigabepositionen radial nach außen von den verriegelten Positionen beim Zusammendrücken der Klemmbereiche (126) aufeinander zu zu bewegen.

2. Obturator (112) nach Anspruch 1, wobei die Obturatorspitze (118) eine Verriegelungsaufnahme (134) und eine Verdrehsicherung (138) umfasst, und wobei der Obturatorkörper (114) eine Verriegelung (136) umfasst, die mit der Verriegelungsaufnahme (134) in Eingriff steht, und wobei der Obturatorkörper (114) eine Verdrehsicherungsaufnahme (140) umfasst, die mit der Verdrehsicherung (138) in Eingriff steht.

3. Obturator (112) nach Anspruch 1 oder 2, wobei jeder der Klemmbereiche (126) eine texturierte Oberfläche (128) umfasst, um eine Betätigung des konformen Mechanismus (124) zu vereinfachen.

4. Obturator (112) nach einem der vorhergehenden Ansprüche, wobei die Obturatorspitze (118) ein Eingriffselement (142) umfasst, das sich in eine Innenbohrung (144) des Obturatorkörpers (114) erstreckt und mit dieser in Eingriff steht, wobei das Eingriffselement (142) eine harte Anschlagfläche (146) umfasst, die einen radial nach außen gerichteten Abschnitt der Innenbohrung (144) blockiert, um Endoskopen einen harten Anschlag zu bieten, die sich innerhalb der Innenbohrung (144) bewegen.

5. Obturator (112) nach Anspruch 4,
- wobei das Eingriffselement (142) dichtend mit der inneren Bohrung (144) des Obturatorkörpers (114) in Eingriff steht, um ein Auslaufen zwischen dem Obturatorkörper (114) und der Obturatorspitze (118) zu verhindern, und / oder
- wobei das Eingriffselement (142) eine vollständige ringförmige Dichtfläche (148) umfasst, die an der inneren Bohrung (144) des Obturatorkörpers (114) abgedichtet ist.

6. Obturator (112) nach einem der Ansprüche 2 bis 5,
- wobei die Verriegelungsaufnahme (134) eine erste Verriegelungsaufnahme ist, wobei die Obturatorspitze (118) eine zweite Verriegelungsaufnahme (134) gegenüber der ersten Verriegelungsaufnahme (134) umfasst, wobei die Verriegelung (136) eine erste Verriegelung ist, wobei der Obturatorkörper (114) eine zweite Verriegelung (136) gegenüber der ersten Verriegelung (136) umfasst, wobei die ersten und zweiten Verriegelungen (136) mit den ersten bzw. zweiten Verriegelungsaufnahmen (134) in Eingriff stehen, und / oder
- wobei die Verdrehsicherung (138) eine erste Verdrehsicherung ist, wobei die Obturatorspitze (118) eine zweite Verdrehsicherung (138) umfasst, die der ersten Verdrehsicherung (138) gegenüberliegt, wobei die Verdrehsicherungsaufnahme (140) eine erste Verdrehsicherungsaufnahme ist, wobei der Obturatorkörper (114) eine zweite Verdrehsicherungsaufnahme (140) gegenüber der ersten Verdrehsicherungsaufnahme (140) umfasst, wobei die ersten und zweiten Verdrehsicherungen (138) mit den ersten bzw. zweiten Verdrehsicherungsaufnahmen (140) in Eingriff stehen.

7. Trokaranordnung (100), umfassend:
- einen Trokar (102) mit einem länglichen rohrförmigen Element (104), das sich zwischen einem distalen Ende (106), das zum Einführen in eine Operationsstelle eingerichtet ist, und einem proximalen Ende mit einem Gehäuse (108) erstreckt, das zum Einführen von chirurgischen Instrumenten in das rohrförmige Element (104) eingerichtet ist, wobei das Gehäuse (108) ein Paar gegenüberliegender Verriegelungsaufnahmen (110) umfasst; und
- einen Obturator (112) nach einem der vorhergehenden Ansprüche, wobei die Verriegelungen (122) des Obturators (112) lösbar an den Verriegelungsaufnahmen (110) des Trokars (102) verriegelt sind, wobei der konforme Mechanismus (124) funktionsmäßig verbunden ist, um die Verriegelungen (122) von den Verriegelungsaufnahmen (110) zu lösen.

8. Trokaranordnung (100) nach Anspruch 7, wobei die Trokarabdeckung (116) eine Verdrehsicherung (130) umfasst, die mit einer Verdrehsicherungsaufnahme in Eingriff steht, die im Gehäuse (108) des Trokars (102) ausgebildet ist, um einer relativen Drehung des Trokars (102) und des Obturators (112) zu widerstehen.

## Revendications

1. Obturateur (112) comprenant :
un corps d'obturateur allongé (114) s'étendant d'un couvercle de trocart (116) à une pointe d'obturateur opposée (118), dans lequel le couvercle de trocart (116) comporte un rebord unique (120) pour s'engager avec un trocart (102), dans lequel le couvercle de trocart (116) comporte une paire de verrous opposés (122) s'étendant radialement vers l'intérieur depuis le rebord (120) et s'étendant distalement depuis celui-ci, dans lequel le couvercle de trocart (116) comporte un mécanisme flexible (124) relié de manière fonctionnelle pour déplacer les verrous (122) entre des positions verrouillées et des positions de libération situées radialement vers l'extérieur par rapport aux positions verrouillées, dans lequel le mécanisme flexible (124) comporte une paire de régions de pincement opposées (126) décalées de manière circonférentielle par rapport aux verrous (122) autour du rebord unique (120), dans lequel le mécanisme flexible (124) est configuré pour déplacer les verrous (122) des positions verrouillées vers les positions de libération situées radialement vers l'extérieur par rapport aux positions verrouillées lors du pincement des régions de pincement (126) l'une vers l'autre.

2. Obturateur (112) selon la revendication 1, dans lequel la pointe d'obturateur (118) comporte un réceptacle de verrou (134) et une clavette anti-rotation (138), et dans lequel le corps d'obturateur (114) comporte un verrou (136) engagé dans le réceptacle de verrou (134), et dans lequel le corps d'obturateur (114) comporte un réceptacle de clavette anti-rotation (140) engagé avec la clavette anti-rotation (138).

3. Obturateur (112) selon la revendication 1 ou 2, dans lequel chacune des régions de pincement (126) comporte une surface texturée (128) pour faciliter l'actionnement du mécanisme flexible (124).

4. Obturateur (112) selon l'une des revendications précédentes, dans lequel la pointe d'obturateur (118) comporte un élément d'engagement (142) s'étendant dans un alésage intérieur (144) du corps d'obturateur (114) et engagé avec celui-ci, dans lequel l'élément d'engagement (142) comporte une surface de butée dure (146) bloquant une partie radialement tournée vers l'extérieur de l'alésage intérieur (144) pour fournir une butée dure aux endoscopes se déplaçant à l'intérieur de l'alésage intérieur (144).

5. Obturateur (112) selon la revendication 4,
- dans lequel l'élément d'engagement (142) est engagé de manière étanche dans l'alésage intérieur (144) du corps d'obturateur (114) pour empêcher les fuites entre le corps d'obturateur (114) et la pointe d'obturateur (118), et/ou
- dans lequel l'élément d'engagement (142) comporte une surface d'étanchéité annulaire complète (148) scellée à l'alésage intérieur (144) du corps d'obturateur (114).

6. Obturateur (112) selon l'une des revendications 2 à 5,
- dans lequel le réceptacle de verrou (134) est un premier réceptacle de verrou, dans lequel la pointe d'obturateur (118) comporte un deuxième réceptacle de verrou (134) opposé au premier réceptacle de verrou (134), dans lequel le verrou (136) est un premier verrou, dans lequel le corps d'obturateur (114) comporte un deuxième verrou (136) opposé au premier verrou (136), dans lequel les premier et deuxième verrous (136) sont engagés dans les premier et deuxième réceptacles de verrous (134), respectivement, et/ou
- dans lequel la clavette anti-rotation (138) est une première clavette anti-rotation, dans lequel la pointe d'obturateur (118) comporte une deuxième clavette anti-rotation (138) opposée à la première clavette anti-rotation (138), dans lequel le réceptacle de clavette anti-rotation (140) est un premier réceptacle de clavette anti-rotation, dans lequel le corps d'obturateur (114) comporte un deuxième réceptacle de clavette anti-rotation (140) opposé au premier réceptacle de clavette anti-rotation (140), dans lequel les premier et deuxième clavettes anti-rotation (138) sont engagées dans les premier et deuxième réceptacles de clavettes anti-rotation (140), respectivement.

7. Ensemble trocart (100) comprenant :
un trocart (102) comportant un élément tubulaire allongé (104) s'étendant entre une extrémité distale (106) configurée pour être insérée dans un site chirurgical et une extrémité proximale comportant un logement (108) configuré pour l'introduction d'instruments chirurgicaux dans l'élément tubulaire (104), dans lequel le logement (108) comporte une paire de réceptacles de verrous opposés (110) ; et
un obturateur (112) selon l'une des revendications précédentes, dans lequel les verrous (122) de l'obturateur (112) sont verrouillés de manière libérable sur les réceptacles de verrous (110) du trocart (102),
dans lequel le mécanisme flexible (124) est relié de manière fonctionnelle pour libérer les verrous (122) des réceptacles de verrous (110).

8. Ensemble trocart (100) selon la revendication 7, dans lequel le couvercle de trocart (116) comporte une clavette anti-rotation (130) engagée dans le réceptacle de clavette anti-rotation (132) défini dans le logement (108) du trocart (102) pour résister à la rotation relative du trocart (102) et de l'obturateur (112).
